# EUROPEAN PATENT APPLICATION

(11) **EP 3 017 791 A1**
(43) Date of publication of application: **11.05.2016**
(21) Application number: 14820324.3
(22) Date of filing: 04.07.2014
(51) Int. Cl.: A61F 2/14, A61F 9/00, A61F 2/76

(54) **NASOLACRIMAL DUCT TUBE INCLUDING LACHRYMAL PASSAGE**

(30) Priority: 05.07.2013 KR 20130078840
(71) Applicant: IUCF-HYU (Industry-University Cooperation Foundation Hanyang University), Seoul 133-791 (KR)
(72) Inventor: LIM, Han Woong, Seoul 138-815 (KR)
(74) Representative: Raynor, Stuart Andrew
(86) International application number: PCT/KR2014/006030
(87) International publication number: WO 2015/002510

(57) **Abstract**

The present invention relates to a nasolacrimal duct tube. A nasolacrimal duct tube, according to one embodiment of the present invention, comprises a lachrymal passage enabling lachrymal fluid to move between the inside and outside of the tube. According to the present invention, since lachrymal fluid can easily be discharged even while the tube is inserted into a human body, it is possible to decrease the possibility of inflammation occurring and the risk of infection, and it is possible to permanently leave the tube in the body without removing the tube after the operation.

## Description

### [Technical Field]

The present invention relates to a nasolacrimal duct tube, and more particularly to a nasolacrimal duct tube that includes a lachrymal passage that allows lachrymal fluid to flow between the inside and the outside of the tube, and a device for insertion of a nasolacrimal duct including the tube.

### [Background Art]

Tears of a human being are produced in a lachrymal gland situated behind the upper eyelid, and flow to a hole called a lachrymal point, are collected in a lacrimal sac, pass through a nasolacrimal duct, and are discharged to a nasal meatus (nose). However, if the nasolacrimal duct is narrowed or blocked, tears are stagnated, making the field of view blurred, making peripheral skin of an eyelid ulcerated, or frequently causing sleeps.

In order to treat the symptom, fistula surgeries, such as a method of inserting a silicon tube in a passage through which tears flow down to widen the nasolacrimal duct that is blocked or narrows and then removing the silicon tube after a predetermined period of time or a method of, when the nasolacrimal duct is completely blocked, creating a new fistula through a nose/lacrimal sac connection operation (dacryocystohinostomy), inserting a silicon tube into the new lachrymal passage, and removing the silicon tube after a predetermined period of time, are conducted.

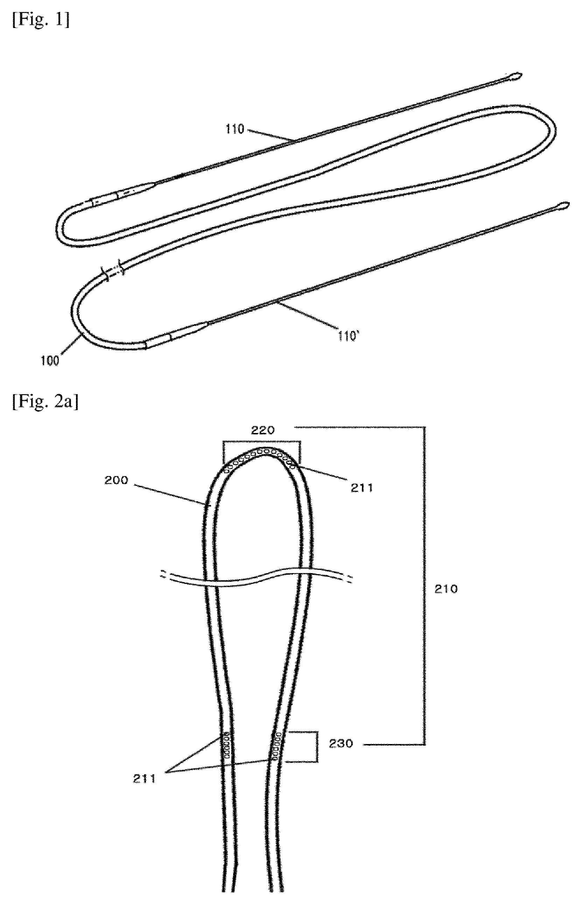

FIG. 1 is a device for insertion of a nasolacrimal duct according to the related art.

In general, as illustrated in FIG. 1, the device for insertion of a nasolacrimal duct used in a fistula surgery has a structure in which rod-shaped metallic probes 110 and 110' are connected to opposite ends of a flexible tube 100. Because the device for insertion of a nasolacrimal duct according to the related art does not have any passage through which tears may be introduced or discharged, tears cannot be properly discharged while the tube is inserted into the human body.

That is, because the tube occupies the greater part of the tear discharge passage while the tube is inserted although the tube is inserted into the human body for at least 3 months or not less than 6 months when the fistula surgery is conducted, the produced tears may be discharged only through a narrow passage between the tube and the lachrymal passage. Accordingly, tears cannot be smoothly discharged and there is a high possibility of inflammation because the tears are easily stagnated, and thus the risk of infection also increases.

### [Disclosure]

### [Technical Problem]

Therefore, the present invention provides a nasolacrimal duct tube that includes a lachrymal passage that allows lachrymal fluid to be easily discharged even while the nasolacrimal duct tube is inserted into a human body, and a device for insertion of a nasolacrimal duct including the tube.

### [Technical Solution]

A nasolacrimal duct tube according to an embodiment of the present invention includes a lachrymal passage provided such that lachrymal fluid is introduced and discharged between the inside and the outside of the tube.

According to an embodiment of the present invention, the lachrymal passage includes a lachrymal fluid introduction part that is formed at a point corresponding to a lachrymal point when the nasolacrimal duct tube is mounted.

According to an embodiment of the present invention, the lachrymal passage further includes a lachrymal fluid discharge part that is formed at a point corresponding to a nasal meatus when the nasolacrimal duct tube is mounted.

According to an embodiment of the present invention, the lachrymal fluid discharge part is formed at a point or points spaced from the lachrymal fluid introduction part in one or opposite directions by a predetermined distance.

According to an embodiment of the present invention, the tube comprises a length adjusting part adjusting the distance of the lachrymal fluid introduction part and the lachrymal fluid discharge part.

A device for insertion of a nasolacrimal duct according to an embodiment of the present invention includes a tube that is flexible and is inserted along the nasolacrimal duct; and a lachrymal passage that is formed in the tube and by which lachrymal fluid is introduced and discharged between the inside and the outside of the tube.

### [Advantageous Effects]

According to the present invention, because lachrymal fluid can be easily discharged even while the nasolacrimal duct tube is inserted into a human body, the possibility of inflammation and the risk of infection can be reduced.

### [Description of the Invention]

FIG. 1 illustrates a device for insertion of a nasolacrimal duct according to the related art.
FIG. 2A illustrates a nasolacrimal duct tube according to an embodiment of the present invention.
FIG. 2B illustrates a cross-section of the nasolacrimal duct tube and a tear passage according to the embodiment of the present invention.
FIG. 3 illustrates a nasolacrimal duct tube according to another embodiment of the present invention.
FIG. 4 is a view illustrating a state in which a nasolacrimal duct tube according to an embodiment of the present invention is inserted into a human body.

### [Best Mode]

Hereinafter, preferred embodiments of the present invention will be described in detail with reference to the accompanying drawings. Here, it is noted that in the accompanying drawings, the same reference numerals denote the same elements. Further, a detailed description of known functions and configurations that may make the essence of the present invention unclear will be omitted.

FIG. 2A illustrates a nasolacrimal duct tube according to an embodiment of the present invention.

Referring to FIG. 2A, the nasolacrimal duct tube according to the embodiment of the present invention may include a lachrymal passage 210 that is formed in a tube 200 such that lachrymal fluid is introduced and discharged between the inside and the outside of the tube. Due to the lachrymal passage 210, lachrymal fluid may be introduced into the interior of the tube 200 through the lachrymal passage 210 formed in the tube 200 and may flow along the tube 200 to the inside of a nose (nasal meatus) even while the tube 200 is inserted into the human body. Accordingly, because tears may be smoothly discharged to the inside of the nose (nasal meatus) through the interior of the tube 200, as well as a narrow passage between the tube 200 and a fistula even while the tube 200 is inserted, the possibility of inflammation and the risk of infection can be reduced.

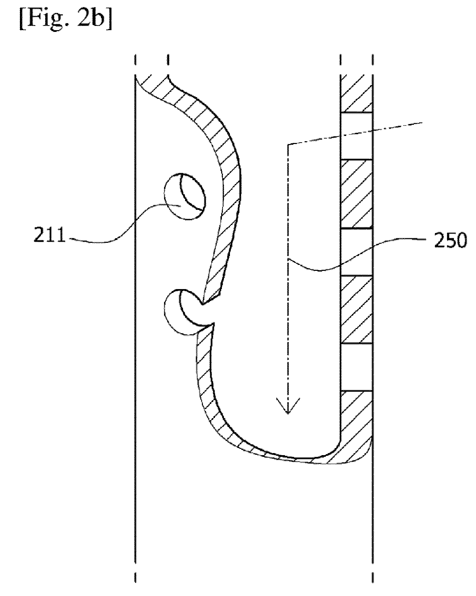

According to the embodiment of the present invention, the lachrymal passage 210 may include one or more openings 211. The number, shape, and size of the openings 211 may be variously selected. FIG. 2B illustrates a cross-section of the nasolacrimal duct tube and a tear passage according to the embodiment of the present invention.

Referring to FIG. 2B, lachrymal fluid may pass through the openings 211 along a tear path 250 and may flow into the interior of the tube 200. The openings 211 may pass through the tube 200 from the outside to the inside of the tube 200 in a direction perpendicular to a surface of the tube 200, or may pass through the tube 200 in an inclined fashion.

According to the embodiment of the present invention, the lachrymal passage 210 may include a lachrymal fluid introduction part 220 that is formed at a point corresponding to a lachrymal point when the nasolacrimal duct tube is mounted. That is, lachrymal fluid produced at the lachrymal point may be smoothly introduced through the lachrymal fluid introduction part 220, by forming the lachrymal fluid introduction part 220 near the lachrymal point when the nasolacrimal duct tube is mounted on the human body. The lachrymal fluid introduced into the lachrymal fluid introduction part 220 may be smoothly discharged through the interior of the tube 200.

According to the embodiment of the present invention, the lachrymal passage 210 may further include a lachrymal fluid discharge part 230 that is formed at a point corresponding to the fistula when the nasolacrimal duct tube is mounted. That is, the lachrymal fluid introduced into the lachrymal fluid introduction part 220 may be smoothly discharged through the lachrymal fluid discharge part 230 situated near the inside of the nose (nasal meatus) through the interior of the tube, by forming the lachrymal fluid discharge part 230 near the inside of the nose (nasal meatus) when the nasolacrimal duct tube is mounted on the human body. Furthermore, the lachrymal fluid may be more smoothly introduced through the lachrymal fluid introduction part 220, by forming a negative pressure in the interior of the tube 200 while the lachrymal fluid is discharged.

According to the embodiment of the present invention, the lachrymal fluid discharge part 230 may be formed at a location or locations spaced apart from the lachrymal fluid introduction part 220 by a predetermined distance towards one or opposite sides of the lachrymal fluid introduction part 220.

The the lachrymal fluid discharge part 230 may be formed at one point spaced apart from the lachrymal fluid introduction part 220 towards one side of the lachrymal fluid introduction part 220 by a predetermined distance, or a pair of lachrymal fluid discharge parts 230 may be formed at points spaced apart from the lachrymal fluid introduction part 220 towards opposite sides of the lachrymal fluid introduction part 220.

The lachrymal fluid discharge part 230 may be formed in the inside of the nose (nasal meatus) spaced apart from the lachrymal fluid introduction part 220 by a predetermined distance situated near the lachrymal point to discharge tears into the inside of the nose (nasal meatus). Accordingly, the predetermined distance may be a distance between the lachrymal point and the inside of the nose (nasal meatus).

According to the embodiment of the present invention, the predetermined distance may be 3 cm to 10 cm, preferably, 4 cm to 6 cm. This is because the distance between a lachrymal point and the inside of a nose (nasal meatus) of a person is generally 3 cm to 10 cm, more specifically, 4 cm to 6 cm although it is somewhat different according to the person.

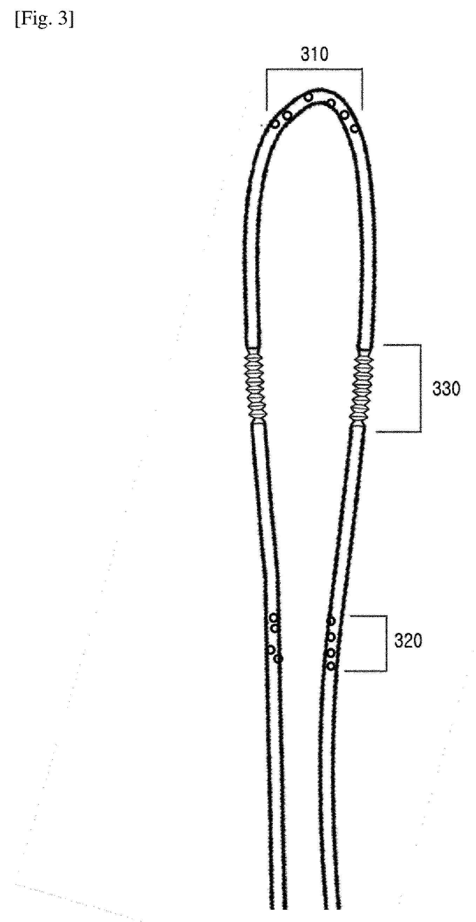

FIG. 3 illustrates a nasolacrimal duct tube according to another embodiment of the present invention.

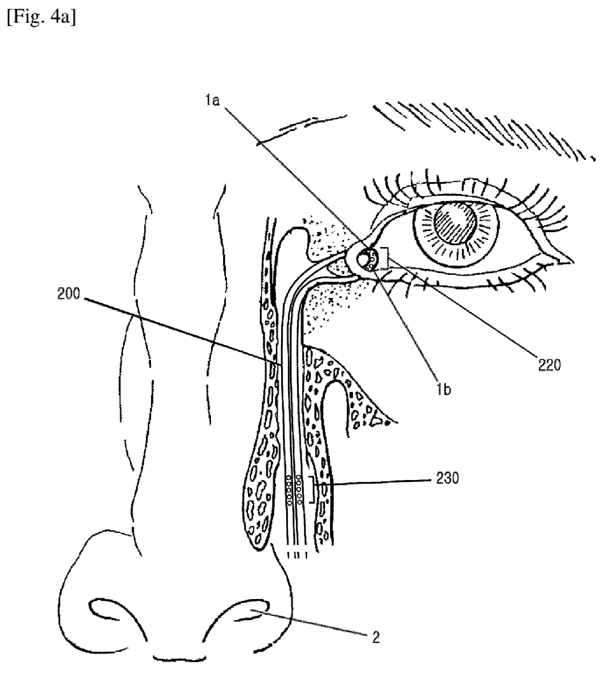

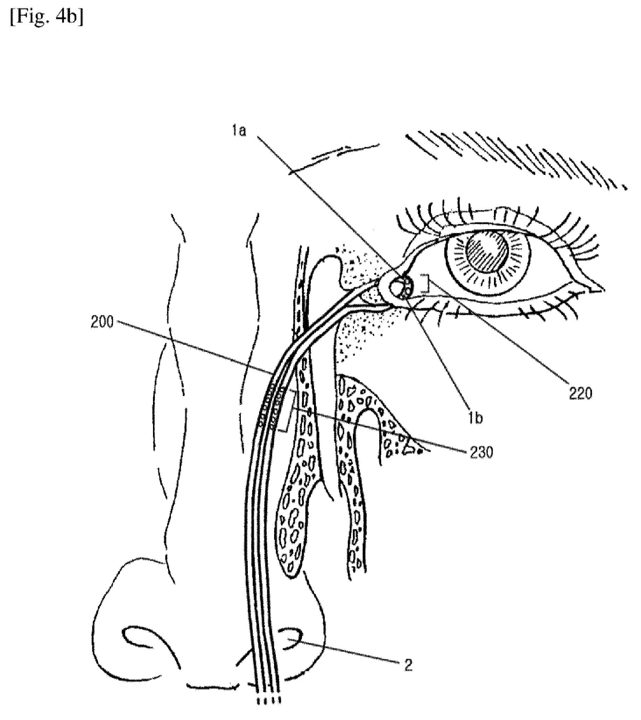

Referring to FIG. 3, the nasolacrimal duct tube 300 according to the embodiment of the present invention may include a length adjusting part 330 by which the distance between a lachrymal fluid introduction part 310 and a lachrymal fluid discharge part 320 may be adjusted. That is, the operator can conveniently select the distance between the lachrymal fluid introduction part 310 and the lachrymal fluid discharge part 320 through the length adjusting part 330 according to the body shape of the patient. The length adjusting part 330 may be implemented by various means. For example, a portion of the tube may be configured in a slidable form such that the upper and lower ends of the tube may be fitted with each other after the tube extends in opposite directions, or a bellows may be formed in the tube such that the length of the tube may be adjusted by the resilient force and the restoring force of the bellows. In the present invention, the length adjusting part 330 is a concept including any structure that functions to adjust the distance between the lachrymal fluid introduction part 310 and the lachrymal fluid discharge part 320, and is not limited in forms. FIG. 4 is a view illustrating a state in which a nasolacrimal duct tube according to an embodiment of the present invention is inserted into a human body. FIG. 4A illustrates a state in which a tube is inserted into a natural fistula of the human body, and FIG. 4B illustrates a state in which a new fistula is created through a nose/lacrimal sac connection operation (dacyocystohinostomy) and the tube is inserted into the new fistula.

The tube according to the embodiment of the present invention may be inserted in the following method.

First, after the probes are inserted through upper and lower lachrymal point 1a and 1b and are extracted to the outside, the tube 200 is inserted from the lachrymal points 1a and 1b to a lower nasal meatus 2 through a guide operation of the probes. Thereafter, when a length adjusting part is present, the location of the tube 200 may be adjusted such that the lachrymal fluid discharge part 230 is located at a portion of the nasal meatus 2 by using the length adjusting part. If necessary, the tube 200 may be fixed so as to be left in the interior of the human body in order to maintain a fluid-communication state of the nasal meatus, by separating the probes from the tube 200 and binding the tip ends of the tube 200 such that a node is formed. The method of mounting a device for insertion of a nasolacrimal duct may also be variously performed in a range that is apparent to those skilled in the art to which the present invention pertains.

According to the present invention, the lachrymal fluid introduction part 220 is formed at a point corresponding to the lachrymal points 1a and 1b such that the lachrymal fluid is introduced into the interior of the tube 200 through the lachrymal fluid introduction part 220, and the lachrymal fluid introduced into the tube 200 flows down through the interior of the tube 200 and then is discharged through the lachrymal fluid discharge part 230. Accordingly, even while the tube 200 is inserted into the human body, the lachrymal fluid can be easily discharged, decreasing the possibility of inflammation and the risk of infection, and can be permanently left in the interior of the human body without separately removing the tube 200 after the surgery.

The material of the flexible tube according to the embodiment of the present invention may be silicon. Furthermore, when a probe or a guide is provided, it is preferable to use a stiff material rather than to use a silicon tube such that the tube may be easily inserted into a lachrymal meatus. For example, the tube may be formed of a material selected from polyolefin, polyamide, polyurethane, or a group thereof.

The device for insertion of a nasolacrimal duct according to the embodiment of the present invention may include a tube that is flexible and is inserted along a nasolacrimal duct, and a lachrymal passage that is formed in the tube such that lachrymal fluid is introduced and discharged between the inside and the outside of the tube.

The device for insertion of a nasolacrimal duct according to the embodiment of the present invention may further include a pair of probes or guides that are flexible and are inserted along the nasolacrimal duct. The tube, the probes, or guide that are flexible and are inserted along the nasolacrimal duct may have various forms in a range that is apparent to those skilled in the art to which the present invention pertains.

The embodiments of the present invention disclosed in the specification and the drawings merely suggest specific examples to easily describe the technical contents of the present invention and help understanding of the present invention, but are not intended to limit the scope of the present invention. it will be apparent to those skilled in the art to which the present invention pertains that other modifications may be made based on the technical spirit of the present invention.

## Claims

1. A nasolacrimal duct tube that is flexible and and is inserted along a nasolacrimal duct, the nasolacrimal duct tube comprising:
a lachrymal passage provided such that lachrymal fluid is introduced and discharged between the inside and the outside of the tube.

2. The nasolacrimal duct tube according to claim 1, wherein the lachrymal passage comprises:
a lachrymal fluid introduction part formed at a point corresponding to a lachrymal point when the nasolacrimal duct tube is mounted.

3. The nasolacrimal duct tube according to claim 2, wherein the lachrymal passage further comprises:
a lachrymal fluid discharge part formed at a point corresponding to a nasal meatus when the nasolacrimal duct tube is mounted.

4. The nasolacrimal duct tube according to claim 3, wherein the lachrymal fluid discharge part is formed at a point or points spaced from the lachrymal fluid introduction part in one or opposite directions by a predetermined distance.

5. The nasolacrimal duct tube according to claim 4, wherein the predetermined distance is 3 cm to 10 cm.

6. The nasolacrimal duct tube according to claim 3, wherein the tube comprises;
a length adjusting part adjusting the distance of the lachrymal fluid introduction part and the lachrymal fluid discharge part.

7. The nasolacrimal duct tube according to claim 1, wherein the lachrymal passage comprises one or more openings.

8. The nasolacrimal duct tube according to claim 1, wherein a material of the tube is silicon.

9. A device for insertion of a nasolacrimal duct comprising:
a tube being flexible and inserted along the nasolacrimal duct; and
a lachrymal passage formed in the tube such that lachrymal fluid is introduced and discharged between the inside and the outside of the tube.
